# EUROPÄISCHE PATENTANMELDUNG

(11) **EP 3 249 406 A1**
(43) Veröffentlichungstag der Anmeldung: **29.11.2017**
(21) Anmeldenummer: 16171730.1
(22) Anmeldetag: 27.05.2016
(51) Int. Cl.: G01N 33/569

(54) **VERFAHREN ZUR BESTIMMUNG DER ANZAHL VON INFEKTIONSHERDEN EINER ZELLKULTUR**

(71) Anmelder: PerkinElmer Cellular Technologies Germany GmbH, 22525 Hamburg (DE)
(72) Erfinder: GARBOW, Norbert, 22589 Hamburg (DE)
(74) Vertreter: dompatent von Kreisler Selting Werner - Partnerschaft von Patent- und Rechtsanwälten mbB

(57) **Zusammenfassung**

Verfahren zur Bestimmung der Anzahl von Infektionsherden einer Zellkultur mit den Schritten: Infizieren einer in einem Probenträger angeordneten Zellkultur mit Viren, Zählen von ggf. infizierten Bereichen der Zellkultur mittels Durchlichtverfahren, Markieren infizierter Zellen mit Fluoreszenzmarkern, Zählen von infizierten Bereichen der Zellkultur mittels Fluoreszenzanalyseverfahren und bereichsweises Auswerten der in beiden Verfahren bestimmten Bereiche zur Bestimmung der Anzahl von Infektionsherden.

## Beschreibung

Die Erfindung betrifft ein Verfahren zur Bestimmung der Anzahl von Infektionsherden einer Zellkultur.

Zur Bestimmung der Anzahl von Infektionsherden einer Zellkultur ist es bekannt, dass zunächst eine Zellkultur auf einem Probenträger gezüchtet wird. Als Probenträger sind insbesondere Titerplatten und Mitkotiterplatten geeignet, wobei in den einzelnen Wells Zellkulturen gezüchtet werden, sodass die Zellen am Wellboden anwachsen. Anschließend erfolgt ein Infizieren der Zellen mit einer viralen Flüssigkeit. Diese ist üblicherweise verdünnt, wobei unterschiedlichen Wells inbesonders virale Flüssigkeiten in unterschiedlicher Verdünnung zugegeben wird. Nach einer vorgegebenen, insbesondere von der Art der Viren abhängigen Zeitspanne, erfolgt ein Entnehmen, insbesondere ein Abpumpen der viralen Flüssigkeit. Die virale Flüssigkeit wirkt üblicherweise mehrere Minuten bis mehrere Stunden auf die Zellkultur ein. Nach Entfernen der viralen Flüssigkeit erfolgt üblicherweise ein Zuführen eines Mediums, das eine Diffusion später freigesetzter Viruspartikel verhindern soll. Mit einem derartigen, insbesondere als Gelschicht eingebrachten Mediums, erfolgt ein Abdecken der Zellkultur. Anschließend erfolgt ein Warten über einen längeren Zeitraum von üblicherweise mehreren Tagen. In diesem Zeitraum sterben die infizierten Zellen ab, platzen und die aus ihnen austretenden Viren breiten sich in die Nachbarzellen aus. Hierdurch wird erzielt, dass verhältnismäßig große Bereiche enstehen, in denen abgestorbene Zellen nicht mehr sichtbar und umgeben von Zonen mit noch lebenden aber bereits infizierte Zellen sind.

Mit Hilfe eines Durchlichtverfahrens können die zerstörten Bereiche bzw. Bereiche, in denen sich keine lebenden Zellen befinden, gezählt werden. Ein derartiges Zählen erfolgt üblicherweise nach Aufnahme eines entsprechenden Bildes durch Personen. Gegebenenfalls kann zur Kontrasterhöhung die Probe mit einem Farbstoff eingefärbt werden. Ein geeigneter Farbstoff wie beispielsweise Methylenblau markiert die lebenden Zellen, sodass die Bereiche, in denen keine lebenden Zellen vorhanden sind, für den Betrachter deutlicher als Löcher zu sehen sind.

Das Bestimmen der Anzahl von Infektionsherden einer Zellkultur mit Hilfe des Durchlichtverfahrens weist insbesondere den Nachteil auf, dass beispielsweise später infizierte Zellen oder Zellen, die auf den Virus anders reagieren, noch nicht abgestorben sind und insofern im Durchlichtverfahren kein entsprechendes Loch gesehen werden kann. Dies gilt beispielsweise auch für Zellen die langsamer reagieren, sodass die entsprechenden Löcher noch sehr klein und insofern nicht oder nur schwer sichtbar sind. Ein weiterer Nachteil des Durchlichtverfahrens besteht darin, dass auch andere Fehlstellen, die nicht von den Viren hervorgerufen wurden, als derartige Fehlstellen erkannt und fälschlicherweise mitgezählt werden. Bei derartigen Fehlstellen kann es sich beispielswese um Bereiche handeln, in denen die Zellen weggeschwemmt wurden oder Verunreinigungen vorhanden sind. Das Wegschwemmen kann beispielsweise bei der Zugabe der viralen Flüssigkeiten oder auch bei der Zugabe des Gels auftreten. Auch die Länge des Zeitraums, die vor einer entsprechenden Durchführung des Durchlichtverfahrens abgewartet werden muss, ist schwer bestimmbar und hängt insbesondere von der Art der Viren ab. Ist der Zeitraum zu kurz gewählt weisen die entsprechenden infizierten Bereiche nur eine geringe Anzahl an abgestorbenen Zellen auf, sodass die entsprechenden Löcher bzw. Fehlstellen schwer zu erkennen sind. Ist der Zeitraum zu lang gewählt wachsen einzelne Bereiche zusammen, sodass schwer bestimmbar ist, ob es sich ursprünglich um einen infizierten Bereich oder um mehrere infizierte Bereiche der Zellkultur handelt.

Ferner ist es zur Bestimmung der Anzahl von Infektionsherden einer Zellkultur bekannt die Zellkultur mit Fluoreszenzmarkern zu behandeln, die die infizierten Zellen markieren. Es ist sodann möglich die infizierten Bereiche zu zählen, da die entsprechenden Bereiche fluoreszieren. Dieses Verfahren hat allerdings den Nachteil, dass nebeneinander liegende infizierte Bereiche nicht voneinander unterschieden werden können und insofern fälschlicherweise nur als ein ursprünglich infizierter Bereich erkannt werden können.

Aufgabe der Erfindung ist es ein Verfahren zur Bestimmung der Anzahl von Infektionsherden einer Zellkultur zu schaffen mit dem die Anzahl der Infektionsherde genauer und zuverlässiger bestimmt werden kann.

Die Lösung der Aufgabe erfolgt erfindungsgemäß durch ein Verfahren gemäß Anspruch 1.

Bei dem erfindungsgemäßen Verfahren zur Bestimmung der Anzahl von Infektionsherden in einer Zellkultur erfolgt zunächst ein Infizieren einer in einem Probenträger angeordneten Zellkultur mit Viren. Dies erfolgt insbesondere durch Zugabe einer viralen Flüssigkeit, die gegebenenfalls verdünnt sein kann. Als Probenträger werden vorzugsweise Titerplatten, insbesondere Mikrotiterplatten verwendet, wobei die Zellkulturen vorzugsweise am Wellboden angewachsen sind. Im nächsten Schritt erfolgt mit Hilfe des bekannten Durchlichtverfahrens ein Bestimmen von gegebenenfalls anfänglich infizierten Bereichen. Mit Hilfe des Durchlichtverfahrens werden somit sämtliche Bereiche bestimmt, in denen keine lebenden Zellen vorhanden sind. Es erfolgt somit ein Bestimmen entsprechender Fehlstellen bzw. Löcher. Hier werden insbesondere nicht nur diejenigen Bereiche bestimmt, in denen Aufgrund des Virus Zellen abgestorben sind, sondern auch diejenigen Bereiche, die beispielsweise durch Verunreinigen oder durch Wegschwemmen keine lebenden Zellen aufweisen.

Ferner erfolgt ein Markieren infizierter Zellen mit einem Fluoreszenzmarker. Die infizierten Bereiche der Zellkultur werden sodann mittels Fluoreszenzanalyseverfahren bestimmt. Hierbei werden zumindest zunächst auch große fluoreszierende Bereiche nur als ein Bereich gezählt.

Anschließend erfolgt die erfindungsgemäße Kombination der beiden Verfahren derart, dass ein bereichsweises Auswerten der in den beiden Verfahren bestimmten Bereiche erfolgt, um die Anzahl der Infektionsherde zu bestimmen. Möglich wäre es beispielsweise die in den beiden Verfahren bestimmten Bereiche aufzusummieren. Da hierbei einzelne Bereiche doppelt gezählt werden, kann auch insofern ein Abgleich der ermittelten Bereiche erfolgen, dass die in beiden Verfahren ermittelten Bereiche nur einfach gezählt werden. Insbesondere können auch in der Fluoreszenzmethode erkannte Bereiche, die in der Durchlichtanalyse mehrere Infektionsherde aufweisen, zuverlässig als multiple Infektionen charakterisiert werden. Hierdurch ist bereits eine erhebliche Verbesserung der Genauigkeit bei der Bestimmung der Anzahl von Infektionsherden einer Zellkultur erzielt.

Bei einer bevorzugten Weiterführung des erfindungsgemäßen Verfahrens erfolgt die Auswertung der mit den beiden Verfahren bestimmten Bereiche derart, dass im Durchlichtverfahren erkannte Bereiche, die jedoch im Fluoreszenzanalyseverfahren nicht erkannt wurden, nicht als Infektionsherd gezählt werden. Durch den Vergleich der mit den beiden Verfahren ermittelten Bereiche ist es möglich Fehlstellen bzw. Löcher zu bestimmten, die nicht durch die virale Infektion hervorgerufen wurden, da derartige Fehlstellen nicht von fluoreszierenden, dass heißt viral infizierten Zellen umgeben sind. Es ist somit mit Hilfe des erfindungsgemäßen Verfahrens möglich Fehlstellen, die beispielsweise durch Wegschwemmen oder Verunreinigung erzeugt wurden, gesondert zu bestimmen, sodass diese das Ergebnis nicht verfälschen. Insbesondere ist es auch möglich diese Bereiche gesondert zu zählen oder auszuwerten. Hierdurch ist eine Qualitätskontrolle der Probe möglich. Beispielsweise kann bei einer vorgegebenen Anzahl an derartigen Störstellen, an denen die Verunreinigungen, Ausschwemmungen oder dergleichen entstanden sind, die entsprechende Probe bei der Auswertung nicht berücksichtigt werden. Dies kann auf Grund der Anzahl oder der Größe der entsprechenden Fehlstellen insbesondere automatisch erfolgen. Die Kontrolle der Häufigkeit und Größe dieser Störstellen ist auch für die Qualitätskontrolle der Probenpräparation von Bedeutung.

Bei einer weiteren bevorzugten Ausführungsform der Erfindung werden Bereiche ermittelt die im Fluoreszenzverfahren, aber nicht im Durchlichtverfahren erkannt werden. Diese Bereiche werden als Infektionsherde der Zellkultur mitgezählt. Hierbei handelt es sich um Bereiche, die virusinfizierte Zellen aufweisen, bei denen jedoch noch keine so große Schädigung aufgetreten ist, dass eine entsprechende Anzahl abgestorbener Zellen, die dann auch zerfallen und als leerer Bereich mit der Durchlichtmethode sichtbar wäre, vorhanden ist. Insbesondere ist es auch möglich diese Bereiche gesondert zu zählen oder auch zu wichten. Zweckmäßig kann diese Analyse z.B. sein, um Hinweise auf stark variierende Infektionsgeschwindigkeit der Viren zu erhalten. Auch eine Qualitätskontrolle bestimmter Aspekte der Probenpräparation ist hiermit möglich.

Bei einer weiteren bevorzugten Ausführungsform der Erfindung erfolgt ein Analysieren der insbesondere in beiden Verfahren erkannten Bereiche bezogen auf deren Größe, Ausdehnung und/oder Fläche. Insbesondere bei Überschreiten einer vorgegebenen Grenzgröße, dass heißt bei Überschreiten einer vorgegebenen Ausdehnung oder einer vorgegebenen Fläche, kann der entsprechende Bereich mehrfach gezählt werden. Hier liegt die Annahme zugrunde, dass es sich um mehrere zusammengewachsene ursprünglich infizierte Bereiche handelt.

Vorzugsweise erfolgt ein genaueres Analysieren derartiger Bereiche. So werden für Bereiche, die im Fluoreszenzanalyseverfahren erkannt werden und eine vorgegebene Grenzgröße überschreiten, in Abhängigkeit der Größe, der Form und oder der Fluoreszenzstärke Anzahlen festgelegt. Desweiteren ist es möglich diese Bereiche insofern näher zu analysieren, dass die Anzahl der Fehlstellen betrachtet wird. Die Anzahl der Fehlstellen, die auf Grund der Fluoreszenz auf abgestorbene Zellen zurückzuführen sind, stellt vorzugsweise die Mindestanzahl der in diesem Bereich ursprünglich infizierten Bereiche dar. Gegebenenfalls wird diese Zahl noch auf Grund der starken Fluoreszenz erhöht, da davon auszugehen ist, dass in diesem Bereich beispielsweise auch ursprünglich infizierte Zellen vorhanden sind, die bisher noch nicht abgestorben sind, sodass keine Fehlstelle entstanden ist.

Vorzugsweise erfolgt ein Festlegen der Grenzgröße in Abhängigkeit der Proben und/oder der Messungen. So ist es beispielsweise möglich die Grenzgröße auf Basis einer Größenverteilung der entsprechenden aufgefundenen Bereiche zu bestimmen. Möglich wäre beispielsweise auch ein Vergleich mit dem MedianWert derartiger aufgefundener Bereiche. Insbesondere kann eine Bestimmung des Grenzwerts auf Basis einer Größenstatistik der unterschiedlichen Bereiche erfolgen.

Besonders bevorzugt ist es, das Durchlichtverfahren oder das Fluoreszenzanalyseverfahren als automatisierte Verfahren durchzuführen. Insbesondere erfolgt dies auf Basis bildgebender Verfahren. Geeignet sind hierfür insbesondere automatisierte Mikroskope oder Plattenlesegeräte mit Bildverarbeitungsverfahren. Geeignet ist hierfür beispielsweise der Plattenreader EnSight des Herstellers PerkinElmer.

Zur Erhöhung des Kontrasts im Durchlichtverfahren kann die Zellkultur eingefärbt werden. Dies kann mit an sich bekannten Verfahren und Farbstoffen, insbesondere Methylenblau erfolgen. Desweiteren kann wie bei Verfahren zur Bestimmung von Infektionsherden einer Zellkultur nach dem Stand der Technik das Anwachsen der Zellen, das Infizieren der Zellen mit viraler Flüssigkeit, das Abdecken mit einem Gel, wie bekannt erfolgen. Insbesondere können die Zellkulturen mittels einer gegebenenfalls verdünnten viralen Flüssigkeit infiziert werden. Bevorzugt ist es hierbei, dass der Verdünnungsfaktor bei der Bestimmung der Anzahl der Infektionsherde berücksichtigt wird. Bei Verwendung von Titerplatten, insbesondere Mikrotiterplatten, ist es desweiteren bevorzugt, dass auf jedem Well dieselbe Zellkultur vorhanden ist und oder dieselben virale Flüssigkeit gegebenenfalls in unterschiedlichen Verdünnungen zugeführt werden. Insbesondere durch die Verwendung von Mikrotiterplatten mit einer großen Anzahl von Wells ist es auch möglich unterschiedliche Bereiche mit unterschiedlichen Zellkulturen oder unterschiedlichen viralen Flüssigkeiten zu nutzen.

Bei einer bevorzugten Weiterbildung der Erfindung werden Kontrollwerte zur Qualitätskontrolle insbesondere automatisch ermittelt. Hierbei kann es sich um eine nähere Betrachtung oder auch nur ein Zählen der Fehlstellen handeln, bei denen es sich um Fehlstellen handelt, die beispielsweise durch Wegschwimmen oder Verunreinigungen entstanden sind und nicht durch abgestorbene Zellen. Wichtige Kontrollwerte sind ferner die Qualität der Objekterkennung, die Textur der insbesondere nicht durch eine Virusinfektion gestörten Zellschichten oder auch die über eine Probe gemittelte Stärke des spezifischen Infektionsmarkers. Desweiteren können auch Details über die Größe oder Morphologie der erkannten Infektionsbereiche für die Interpretation der Messung herangezogen werden.

Anstelle eines Durchlichtverfahrens können auch andere unspezifische Erkennungsverfahren eingesetzt werden. Geeignet sind hierfür auch Fluoreszenzverfahren, die Zellen oder Bereiche von Zellen unabhängig von einer Infektion markieren und die dann mikroskopisch ausgelesen werden. Um eine einfache Unterscheidung von der infektionsspezifischen Markierung zu erlauben wird hierfür ein Fluoreszenzfarbstoff mit unterschiedlichen Eigenschaften (wie z.b. der Emissionswellenlänge) bevorzugt.

Ebenso ist es möglich anstelle eines Fluoreszenzanalyseverfahrens infektionsspezifische Erkennungsverfahren einzusetzen. In diesen werden nichtfluoreszente infektionsspezifische Marker verwendet. Hierbei kann es sich beispielsweise um Farbstoffe für kolorimetrische Verfahren handeln.

Bei geeigneten Zelltypen kann es auch möglich sein, Bereiche infizierter Zellen ohne speziellen Marker anhand der Textureigenschaften von nicht infizierten Zellbereichen zu unterscheiden

Ferner ist es möglich, dieses Verfahren nicht nur für die Untersuchung der Infektion von Zellenkulturen durch Viren zu nutzen. Denkbar sind beispielsweise auch Bakterien, die Zellkulturen infizieren oder Zellen bzw. (einzellige) Lebewesen wie etwa Amöben, die Bakterienkulturen fressen.

In der anliegenden Skizze ist als schematisches Beispiel ein Well einer Titerplatte dargestellt. In dem Bereich innerhalb des Wellrandes ist eine Zellkultur angeordnet, die wie vorstehend beschrieben mit Viren infiziert wurde. Hierbei bilden sich unterschiedliche Bereiche aus. Der weiß dargestellte Bereich ist ein Bereich in dem Zellen beispielsweise bei der Zugabe des viralen Fluids oder dergleichen weggespült wurden und insofern ein Bereich ohne Zellen entsteht. Die dunkel schattierten Bereiche sind fluoreszierende Bereiche, dass heißt Bereiche in denen infizierte Zellen vorhanden sind. Hierbei können sich unterschiedliche Bereiche ausbilden. Im dargestellten Beispiel ist es ein Bereich ohne freies Zentrum. Hierbei handelt es sich um einen Bereich in dem zwar Zellen infiziert sind, jedoch noch kein freies Zentrum abgestorbener Zellen entstanden ist.

Im oberen Bereich der Skizze ist ein infizierter Bereich dargestellt, der ein freies oder leeres Zentrum hat. Das leere Zentrum ist der Bereich in dem abgestorbene Zellen vorhanden sind. Diese sind von infizierten und daher fluoreszierenden Zellen umgeben. Der im linken Bereich des Wells dargestellte große Bereich stellt einen zusammengewachsenen Infektionsherd dar. Dieser weist vier leere Zentren auf, die von fluoreszierenden und somit infizierten Zellen umgeben sind.

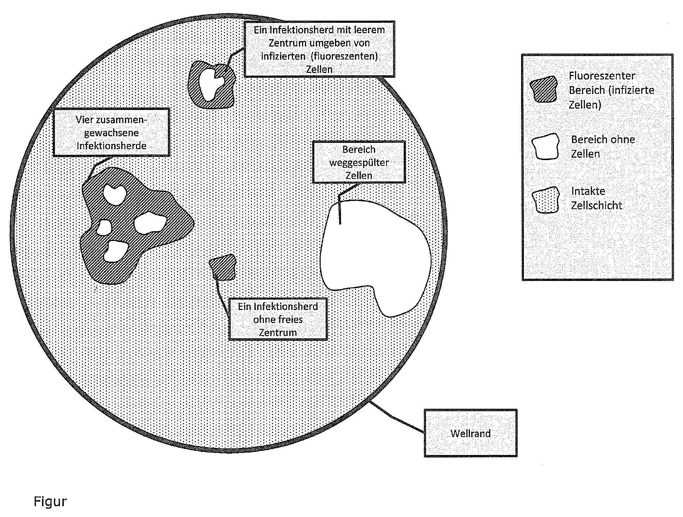

## Patentansprüche

1. Verfahren zur Bestimmung der Anzahl von Infektionsherden einer Zellkultur mit den Schritten
Infizieren einer in einem Probenträger angeordneten Zellkultur mit Viren,
Zählen von ggf. infizierten Bereichen der Zellkultur mittels Durchlichtverfahren,
Markieren infizierter Zellen mit Fluoreszenzmarkern,
Zählen von infizierten Bereichen der Zellkultur mittels Fluoreszenzanalyseverfahren und
bereichsweises Auswerten der in beiden Verfahren bestimmten Bereiche zur Bestimmung der Anzahl von Infektionsherden.

2. Verfahren nach Anspruch 1, wobei Bereiche, die im Durchlichtverfahren, aber nicht im Fluoreszenzanalyseverfahren erkannt werden, nicht gezählt werden.

3. Verfahren nach Anspruch 1 oder 2, wobei Bereiche, die im Fluoreszenzanalyseverfahren aber nicht im Durchlichtverfahren erkannt werden, gezählt werden.

4. Verfahren nach einem der Ansprüche 1 - 3, wobei Bereiche, die im Fluoreszenzanalyseverfahren aber nicht im Durchlichtverfahren erkannt werden, zusätzlich gesondert gezählt werden.

5. Verfahren nach einem der Ansprüche 1 - 4, wobei erkannte Bereiche, die eine vorgegebene Grenzgröße überschreiten, mehrfach gezählt werden.

6. Verfahren nach einem der Ansprüche 1 - 5, wobei für Bereiche, die in dem Fluoreszenzanalyseverfahren erkannt werden und eine vorgegebene Grenzgröße überschreiten, in Abhängigkeit der Größe, Form und/oder der Fluoreszenzstärke eine Anzahl festgelegt wird.

7. Verfahren nach einem der Ansprüche 1 - 6, wobei Bereiche, die im Fluoreszenzanalyseverfahren erkannt werden und eine vorgegebene Grenzgröße überschreiten, mit dem entsprechenden Bereich des im Durchlichtverfahren ermittelten Bereich verglichen werden und zumindest die Anzahl der in diesem Bereich erkannten Fehlstellen gezählt wird.

8. Verfahren nach einem der Ansprüche 5 - 7, wobei die Grenzgröße in Abhängigkeit der Proben und/oder der Messungen insbesondere automatisch angepasst wird.

9. Verfahren nach einem der Ansprüche 1 - 8, wobei es sich beim Durchlichtverfahren und/oder beim Fluoreszenzanalyseverfahren um automatisch durchgeführte Verfahren, insbesondere auf Basis von bildgebenden Verfahren, handelt.

10. Verfahren nach einem der Ansprüche 1 - 9, wobei die Zellkulturen vor dem Durchführen des Durchlichtverfahrens eingefärbt werden.

11. Verfahren nach einem der Ansprüche 1 - 10, wobei Kontrollwerte zur Qualitätskontrolle insbesondere automatisch ermittelt werden.

12. Verfahren nach einem der Ansprüche 1 - 11, wobei die Zellkultur mittels einer viralen Flüssigkeit, ggf. verdünnt, infiziert wird.

13. Verfahren nach Anspruch 12, wobei der Verdünnungsfaktur bei der Bestimmung der Anzahl der Infektionsherde berücksichtigt wird.

14. Verfahren nach einem der Ansprüche 1 - 13, wobei als Probenträger eine Titerplatte, insbesondere eine Mikro-Titerplatte, verwendet wird.

15. Verfahren nach Anspruch 14, wobei je well der Titerplatte und insbesondere der Mikrotiterplatte dieselbe Zellkultur vorhanden ist.

16. Verfahren nach einem der Ansprüche 13 - 15, wobei die virale Flüssigkeit nach einer Einwirkzeit entfernt und durch eine die Diffusion später freigesetzter Viruspartikel behindernde Abdeckung, etwa eine Gelschicht ersetzt wird.

17. Verfahren nach einem der Ansprüche 1 - 16, wobei die Durchführung der Verfahren nach einer Inkubationszeit von vorzugsweise einigen Minuten und insbesondere mehreren Tagen erfolgt.
